# EUROPEAN PATENT SPECIFICATION

(11) **EP 3 372 647 B1**
(45) Date of publication and mention of the grant of the patent: **30.10.2019**
(21) Application number: 18168289.9
(22) Date of filing: 03.02.2014
(51) Int. Cl.: C09D 5/00, C08L 89/00, C08K 3/08

(54) **HYBRID NANOCOMPOSITE MATERIALS OF AMYLOID FIBRILS AND GOLD**
HYBRID-NANOVERBUNDSTOFFE AUS AMYLOIDFIBRILLEN UND GOLD
MATÉRIAUX NANOCOMPOSITES HYBRIDES DE FIBRILLES AMYLOÏDES ET D'OR

(30) Priority: 12.02.2013 EP 13000719
(43) Date of publication of application: 12.09.2018
(62) Divisional of application: 14704276.6
(73) Proprietor: ETH Zurich, 8092 Zürich (CH)
(72) Inventor: Mezzenga, Raffaele, 8604 Volketswil (CH); Bolisetty, Sreenath, 8051 Zürich (CH)
(74) Representative: Grimm, Siegfried

(56) References cited:
- WO-A2-00/17328
- WO-A2-02/28507
- SREENATH BOLISETTY ET AL: "Amyloid-mediated synthesis of giant, fluorescent, gold single crystals and their hybrid sandwiched composites driven by liquid crystalline interactions", JOURNAL OF COLLOID AND INTERFACE SCIENCE, vol. 361, no. 1, 17 May 2011 (2011-05-17), pages 90-96, XP055066466, ISSN: 0021-9797, DOI: 10.1016/j.jcis.2011.05.018
- SCHEIBEL T ET AL: "CONDUCTING NANOWIRES BUILT BY CONTROLLED SELF-ASSEMBLY OF AMYLOID FIBERS AND SELECTIVE METAL DEPOSITION", PROCEEDINGS OF THE NATIONAL ACADEMY OF SCIENCES, NATIONAL ACADEMY OF SCIENCES, US, vol. 100, no. 8, 15 April 2003 (2003-04-15), pages 4527-4532, XP001180754, ISSN: 0027-8424, DOI: 10.1073/PNAS.0431081100
- BASTUS ET AL: "Gold nanoparticles for selective and remote heating of beta-amyloid protein aggregates", MATERIALS SCIENCE AND ENGINEERING C, ELSEVIER SCIENCE S.A, CH, vol. 27, no. 5-8, 8 August 2007 (2007-08-08), pages 1236-1240, XP022190479, ISSN: 0928-4931, DOI: 10.1016/J.MSEC.2006.08.003
- IVONNE OLMEDO ET AL: "How Changes in the Sequence of the Peptide CLPFFD-NH 2 Can Modify the Conjugation and Stability of Gold Nanoparticles and Their Affinity for [beta]-Amyloid Fibrils", BIOCONJUGATE CHEMISTRY, vol. 19, no. 6, 30 May 2008 (2008-05-30), pages 1154-1163, XP055048828, ISSN: 1043-1802, DOI: 10.1021/bc800016y
- DAEKYUN LEE ET AL: "Photoconductivity of Pea-Pod-Type Chains of Gold Nanoparticles Encapsulated within Dielectric Amyloid Protein Nanofibrils of [alpha]-Synuclein", ANGEWANDTE CHEMIE INTERNATIONAL EDITION, vol. 50, no. 6, 7 February 2011 (2011-02-07), pages 1332-1337, XP055012130, ISSN: 1433-7851, DOI: 10.1002/anie.201004301

## Description

The present invention relates to novel composite materials consisting of amyloid fibrils and elemental gold; to environmentally friendly ("green") methods for manufacturing such materials; to articles comprising such materials; as well as to the use of such materials.

It is known that gold has many industrial and commercial applications. First, Gold stimulates an ever-lasting craze not only in jewelry and decoration markets. Second, due to its combination of exceptional physical and chemical stability, unusual optical properties, catalytic and conductive properties, gold also attracts great interests in many different fields of science and technology. For example, nanoscale gold objects (such as particles, rods and platelets can serve a wide range of applications in catalysts, sensors and optoelectronic devices.

CN20111146409 discloses a method for preparing polypyrrole/gold nano composites. The nanocomposites obtained by this method have inferior properties when compared to the inventive nanocomposites; also the manufacturing is considered disadvantageous, due to the need of polypryrroles and surfactants and it is not considered "green".

CN20101274005 discloses a modification method for gold nanorods, which is characterized in that the gold nanorods are contacted with a DNA solution or a protein antigen solution, the surface of the gold nanorods carries a positive charge. The invention also relates to a gold nanorods-functional molecule composite obtained by the modification method. The composites obtained by this method have inferior properties when compared to the inventive nanocomposites; also the manufacturing is considered disadvantageous as very specific starting materials are used, unsuitable for most commercial applications.

Bolisetty et al (Journal of Colloid and Interface Science2011,90-96, describe self-assembled protein-gold composites in solution. The document discloses the single crystal structure of gold, but does not teach or suggest how to obtain self-supporting or solid materials and consequently does not provide information on its appearance in the solid, dry state.

A number of authors describe composite materials comprising amyloid fibrils and elemental gold: Scheibel et al. (PNAS, 2003 4527-4532); Bamdad (WO02/28507); Bastus et al. (Material Science and Engineering, 2007, 1236-1240); Olmedo et al. (Bioconjugate Chem, 2008, 1154-1163); Lee et al. (Angew. Chem. Int. Ed., 2011, 1332-1337); Dobson et al (WO00/17328). All these authors fail in providing gold in the form of single crystals; making it unsuitable - or at least less suitable - for the applications described in the present invention.

Hence, it is the object of the present invention to provide improved composite materials and to provide improved methods for manufacturing such materials which also allow targeting new applications.

These objectives are achieved by the composite material as defined in claim 1 and the manufacturing method as defined in claim 6. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

As used herein, the terms "a", "an,", "the" and similar terms used in the context of the present invention (especially in the context of the claims) are to be construed to cover both the singular and plural unless otherwise indicated herein or clearly contradicted by the context.

The present invention will be better understood by reference to the **figures.**

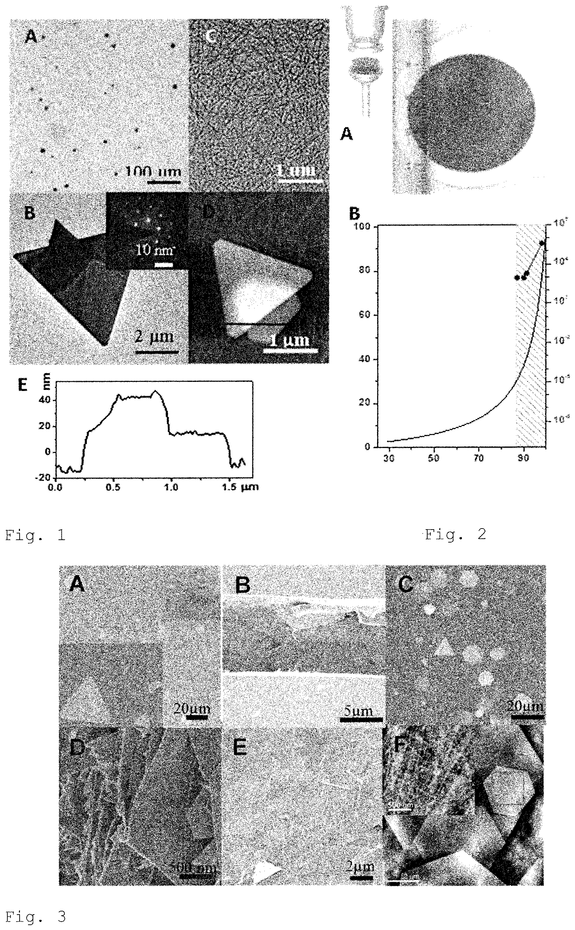

**Figure 1** shows microscopic characterization of gold nanoplatelets and amyloid fibrils in the colloidal suspension. (a) Optical microscopy image of gold single crystal nanoplatelets with high colloid stability. b) TEM image of gold single crystal nanoplatelets. The inset gives the typical single crystal diffraction pattern observed by selected area electron diffraction. (c) TEM image of amyloid fibrils in the suspension. (d) AFM image of a typical gold single crystal plate. (e) The height profile along the line in Figure 1d. A plate thickness of ∼ 25 nm is indicated.
**Figure 2** shows hybrid film fabrication and corresponding electrical conductivity. (a) Filtration setup and a typical hybrid film obtained. (b) Non-linear relationship between the weight (X-axis, unit: %) and volume composition of gold platelets (Left Y-axis, unit: %) in the hybrid films and in-plane conductivity (right Y-axis, unit: S/cm) versus composition. The conductive regime is identified as the striped region; the white region identifies insulating films.
**Figure 3** shows structural characterization of hybrid films with varying gold compositions. SEM images of the hybrid films: (a) Surface and (b) fracture section with 29 wt. % gold. (c) Surface and (d) fracture section with 47 wt. % gold. (e) Surface with 87 wt. % gold. (f) AFM image of the hybrid film surface with 87 wt. % gold. The zoomed-in square regions were shown as the insets.

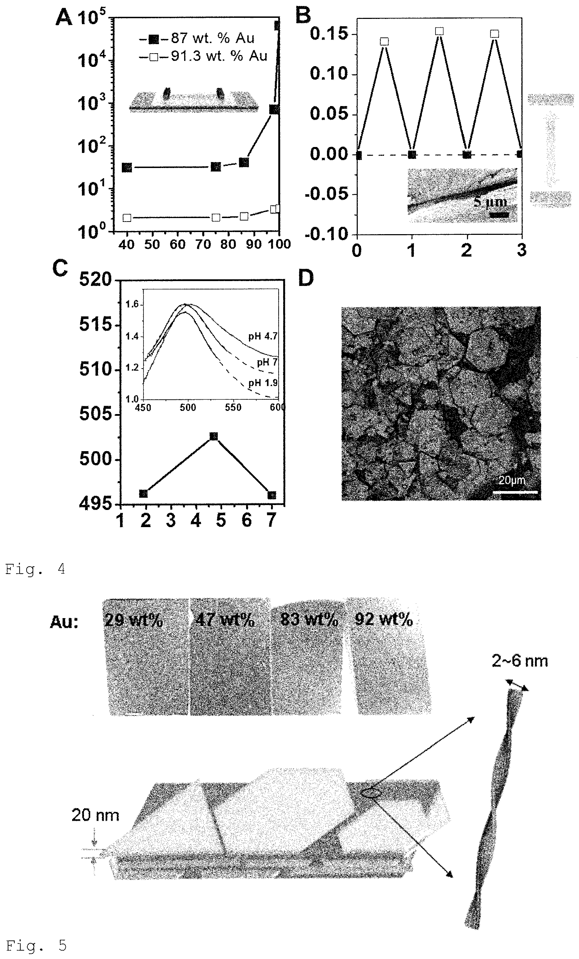

**Figure 4** shows physical properties of the hybrid films. (a) Relative humidity-dependence (x-axis, unit: %) of the resistance (y-axis, unit: Ω) for the films with different gold compositions. The setup is shown as the inset. (b) Humidity-controlled "ON" (open symbol) and "OFF" (solid symbol) states within three dry-wet-dry cycles (X-axis) for the film with 90 wt. % gold. Y-axis is the inverse resistance (unit: 1/Ω). The inset SEM image gives a typical micro-scratch. (c) pH-dependence (x-axis) of the maximum of scattered surface plasmon resonance for the film with 83 wt.% gold. y-axis is the wavelength (unit: nm). The surface plasmon resonance scattering spectra are shown as the inset (scattering intensity/a.u vs. wavelength/nm). (d) Confocal optical microscopy image of the hybrid film with 87 wt. % gold. The exiting wavelength was 488 nm and the signal was collected within the wavelength range of 518-561 nm.
**Figure 5** shows schematic representation of the inventive composites, and the visual appearance thereof with a gold content ranging from 29-92 wt%.

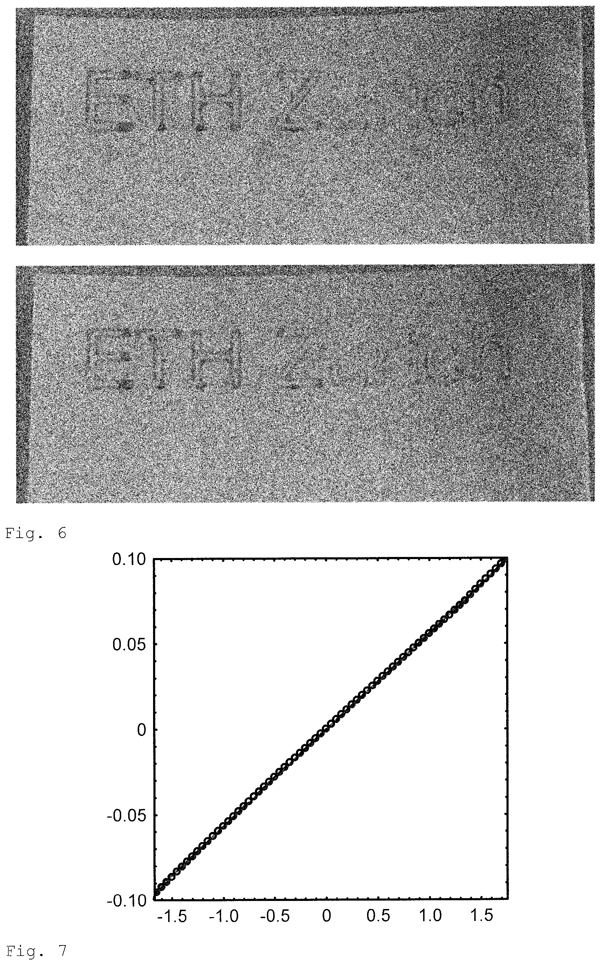

**Figure 6** shows composite materials printed with an ink jet printer as described in ex. 3. Top: letters "ETH Zurich" on paper; bottom: letters "ETH Zurich" on glass.
**Figure 7** shows the IV curve (after annealing) of a composite obtained by ink printing according to ex. 3; voltage (V) on the X-axis and current (A) on the Y-axis.

In a **first aspect,** the present invention relates to composite materials, showing a golden shining and consisting of amyloid fibrils and elemental gold, both as further defined in claim 1. This aspect of the invention shall be explained in further detail below:
The invention relates to composite materials made of inorganic gold platelets and protein fibrils, which are analogous to their natural counterparts selected by evolution and optimized by nature, such as bone, enamel and nacre. In particular, super large and high aspect ratio gold single crystal platelets are combined with protein amyloid fibrils to design a new class of composite materials.

**Amyloid Fibrils:** The term "amyloid fibrils" is generally known in the field and particularly describes fibrils made by proteins or peptides prevalently found in beta-sheet secondary structure. Accordingly, the term amyloid fibrils excludes native proteins.

Without being bound to theory, the roles played by amyloid fibrils are believed to be multiple: they allow reduction of gold salts into platelets, their colloidal stabilization, their tight packing and adhesion and consolidate the platelets into compact films. Advantageously, the amyloid fibrils have high aspect ratio, preferably with ≤ 10 nm in diameter and ≥ 1µm in length. Advantageously, the amyloid fibrils have a highly charged surface. The term highly charged surfaces is generally known in the field and particularly describes surfaces showing electrophoretic mobilities of the order 2 µm·cm/V·s at pH 4.

**Elemental Gold:** The inventive composites comprise gold in elemental form, i.e. oxidation state +/-0. Advantageously, the elemental gold is present in the form of gold platelets, preferably single crystal gold platelets. Such platelets have a high aspect ratio, such as 500: 1, preferably 800:1; typical sizes are up to 20 µm and the thickness only of 100 nm or less, such as 25 nm or less. Without being bound to theory, it is believed that the high aspect ratio gold single crystals provide metal conductivity, fluorescence and plasmonic features. The resulting materials have well-defined layered hierarchical structures and combine physical properties from both individual constituents, such as, for example, water-responsive and tunable conductivities from insulating to metallic levels.

The amount of elemental gold may vary over a broad range, depending on the intended use of the inventive composites. Typically, elemental gold amounts to 10-99 wt.%, preferably 30 - 99 wt.% of the total weight of the composite material. Accordingly, composite materials, of the present invention may have a gold content of 9ct, 14ct, 18ct 21ct, 21.6ct, or 22ct, for example.

**Composite Materials:** The present invention relates in general to a new class of multi-functional, hybrid, nano-sized composites. According to the invention, the hybrid composites are made of 2-dimensional and 1-dimensional nanoscale building blocks, which are well organized into layered hierarchical structure. Single crystal gold nanoplatelets form 2D- building blocks, and amyloid fibrils form 1D-building blocks, see e.g. fig. 1c and 5.

The size of the nano-sized composite materials may vary; typically a range of 20 - 1000 nanometers is found. Without being bound to theory, it is believed this particle size contributes to its golden shiny properties, making it suitable for the applications outlined herein.

The term "hybrid composite" or "hybrid material" refers to materials comprising both, organic components as well as inorganic components in intimate contact. These hybrid composite materials may be present in various forms.

In a **second aspect,** the invention also relates to a method of manufacturing the inventive composite materials. This aspect of the invention shall be explained in further detail below:
The starting materials, amyloid fibrils and gold platelets are known materials. According to the inventive method, the two components were combined into a novel family of layered hybrid films via a simple vacuum-filtration process. To this end, amyloid fibrils offered several advantages over native proteins. Firstly, they allowed reducing the gold salts into colloidal nanoplatelets of very large lateral sizes (∼10¹ µm) and provided the platelets with high colloidal stability. Secondly, their exceptional adhesive properties enabled the assembly of individual platelets into homogeneous layered composites (Mostaert, A. S. et al. Nanotechnology. 2007, 18, 044010; Mostaert, A. S. et al. The Journal of Adhesion 2009, 85, 465-483). Thirdly, their remarkable stiffness and high aspect ratio, allowed them to withstand the filtration process through the vacuum membrane with only negligible losses of the organic component. By using a combination of structural and physical characterization techniques, it was confirmed that well-organized layered hierarchical structures are provided. The inventive composite materials obtained by the process show remarkable properties that none of the constituents could generate alone.

It is considered particularly advantageous that the entire manufacturing is green and eco-friendly.

It is further considered particularly advantageous that the obtained materials have unique optical properties, such as fluorescent and optic-grade golden colour.

Accordingly, the invention provides a process for manufacturing composite materials as described herein, comprising the steps of (a) Growing protein amyloid fibrils, preferably from β-lactoglobulin or lysozyme; (b) Growing single crystal platelets, preferably from chloroauric acid, in the presence of amyloid fibrils; (c) Optionally concentrating the thus obtained single crystal gold platelets in suspensions; and then further processing the thus obtained concentrated suspension of gold platelets-protein fibrils by (d) casting techniques. Accordingly, steps (a) to (c) relate to the manufacturing of the composite materials as described herein, where step (c) is optional and depends on the further processing. These composite materials may then be further processed to obtain self-supporting thin films or coatings on a substrate by method known per se, such as steps (d).

The inventive process may be accomplished by further steps, e.g. preceding step (a) or following step (d), including purification, further processing, assembling and other process steps known to the skilled person.

**Step (a):** The synthesis of amyloid fibrils is a known technology. Suitable is in particular protein hydrolysis followed by β-sheets driven fibrillation, as described e.g. in Jung et al. (Biomacromolecules. 2008, 9, 2477-2486). Suitable starting materials are food-grade proteins, which are structural stable, wide accessible and inexpensive. Such starting materials allow preparation of amyloid fibrils, such as *β*-lactoglobulin. Suitable proteins may be selected from the group consisting of *β*-lactoglobulin, lysozyme, ovalbumin, and serum albumines.

The self-assembly process is facile and controllable. Typical process parameters include incubating protein solution (e.g. 2 wt.% *β*-lactoglobulin) for a prolonged period of time (e.g. 6 h) under acidic conditions (e.g. pH ∼ 2), low ionic strength (e.g. I ≤ 20 mM), high temperature (e.g. T ∼ 90 °C).

**Step (b):** The synthesis of single crystal gold platelets is a known technology. Suitable is in particular the green chemistry method of Bolisetty et al. (Journal of Colloid and Interface Science. 2011, 361, 90-96).

The inventors have produced single crystal gold platelets with super large size (eg. ∼10⁴ nm) and high aspect ratio (up to 10³) using a simple green method. Under controlled conditions (particularly pH, temperature, amyloid fibril concentration), amyloid fibrils can act both as a green reducing agent and a stabilization agent to synthesize the gold platelets and to provide high colloidal stability. In other terms, due to the three-fold roles played by the protein fibrils, only two materials were involved in the fabrication of these unique gold platelets as well as the inventive composites.

Typical process parameters include mixing 0.67 wt. % amyloid fibrils of step a) and 0.066 wt.% chloroauric acid, and then incubating at pH 2 at elevated temperatures (e.g. 60 °C) for a prolonged period of time (e.g. 12 h).

**Step (c):** Non-reacted starting materials may be separated, thereby concentrating the inventive composites in suspension. This may be done by simple centrifugation, and discharging / recycling the non-reacted supernantant aqueous amyloid phase. This step improves product quality.

**Step (d):** To obtain coated substrates or self-supporting thin films, casting techniques may be used. Accordingly, the invention provides for a method, comprising the step of casting a suspension comprising the hybrid composites as described herein. Suitable casting techniques are known in the field and include slow evaporation and doctor-blading techniques.

In a further embodiment, the invention also provides for a composite material obtainable by the method described herein, or obtained according to the method as described herein.

In this embodiment, the amyloid fibrils are preferably prepared from food-grade proteins; preferably selected from the group consisting of *β*-lactoglobulin, lysozyme, ovalbumin, and serum albumines. It is considered particularly advantageous that broadly available, inexpensive food-grade proteins are suitable starting materials for manufacturing the inventive composites. Further, in this embodiment the single crystal gold platelets are simply prepared by reducing gold salts in an aqueous solution optionally further stabilized with *β-*lactoglobulin amyloid fibrils in colloidal state.

It is considered particularly advantageous using such green chemistry for manufacturing elemental gold.

In a **third aspect,** the invention also relates to various uses of the inventive composite materials and to corresponding articles. As outlined above, the present invention provides a new class of multi-functional composite films made of 2D and 1D nanoscale building blocks: single crystal gold nanoplatelets and amyloid fibrils, making it useful in a very broad range of applications. Accordingly, films of the inventive composites can find applications in sensors, diagnostic devices, printed matter (printed paper), printed electronics, micromechanical devices and biological devices. This aspect of the invention shall be explained in further detail below:
In one embodiment, the inventive composites may be used in **electric devices** as conducting materials. The amount of gold in the hybrid composite may be adjusted within virtually the entire composition range (e.g. from 1∼99 wt.%). Therefore, the hybrid composite is insulating when having a low gold content or metal conductive when having a high gold content. By changing the gold nanoplatelets content, the metal conductive window may be tuned within 10² ∼ 10⁵ S/cm.

In one further embodiment, the inventive composites may be used for **decoration, packaging and surface coating.** The hybrid composites of the present invention have the surfaces of homogenous metallic gold color, even at very low gold content of gold (e.g. 29 wt.%). All the composites produced according to this invention have smooth and shiny surfaces. This is attributed to the super-large size and nanoscale thickness of gold platelets as well as their super flat surfaces.

In one further embodiment, the inventive composites may be used as **actuator to control air humidity.** Due to the presence of amyloid fibrils in the hybrid composites, water can diffuse easily within. For example, the composite with 87 wt. % gold has a dependence of the conductivity on the relative humidity. The resistance increased in up to 2 orders of magnitude with the relative humidity over 85 %. By micro-scratching the film with 90 wt. % gold, upon cyclic changes in humidity, the hybrid film may exhibit "ON" and "OFF" conductivity states. Upon exposure to water, the scratched film swells, anneals and shows a resistance close to that of the unscratched film. Accordingly, the inventive composites may be used for sensors responsive to water.

Gold platelets in the composite materials may also have the change of surface plasmon resonance upon exposure to water and the pH changes. For example, due to the lower reflective index of water compared with protein, the resonance peak of gold platelets undergoes a blue shift and becomes more pronounced upon exposure of the film (29 wt. % gold) to water. This resonance peak also varies by changing the pH of the rinsing solution, due to the existence of the isoelectric point of β-lactoglobulin (pH 5.1) as well as stronger optical coupling between gold platelets. Accordingly, the inventive composites may be used for **sensors** responsive to pH change.

The hybrid composites in accordance with the present invention may also be fluorescent.

In a further embodiment, the invention provides an article comprising a substrate and a coating, said coating consisting of an inventive composite material as described herein.

Such articles include decorative articles, such as packaging materials, decorative articles which are partly or fully coated with the inventive composite or which are printed with an ink comprising the inventive composite. Such articles further include electrical devices comprising wires, microdevices or electrical conductors made of the inventive composites.

Such articles further include sensors comprising the inventive composites as functional layer or functional element, particularly for sensing pH, or humidity.

To further illustrate the invention, the following **examples** are provided. These examples are provided with no intend to limit the scope of the invention.

### 1. Manufacturing of hybrid composites by filtration

Step a, Growing protein amyloid fibrils from β-lactoglobulin:
▪ Dissolve β-lactoglobulin into MilliQ water and tune the solution to 2 wt.% and pH 2.
▪ Incubate the solution at 90 °C with slowly stirring for at least 5 h.

Step b, Growing single crystal platelets from chloroauric acid with amyloid fibrils:
▪ Tune the *β*-lactoglobulin fibril solution of step a) to 0.67 wt. % and pH 2.
▪ Drop 2ml 0.1 M HAuCl₄ solution into 60 ml *β*-lactoglobulin fibril solution.
▪ Keep the suspension at 60 °C in an oil bath without stirring for 10 h.

Step c, Concentrating single crystal gold platelets in suspensions:
▪ Centrifuge the suspension of step b) at 3000 r.p.m. for 5 min.
▪ Remove a portion of supernatant solution, which contains ≈100 % amyloid fibrils.
▪ Stir slowly the remaining part to get a homogeneous suspension comprising the inventive hybrid nanocomposites.

Step d, Filtrating the gold platelets-protein fibrils suspension:
▪ Filter the suspension with the vacuum (100mBar) with a vacuum filtration assembly and Nylon filtration membranes (Pore size of 0.2 pm).
▪ Air-dry the hybrid composite at a relative humidity content of ∼40 % for at least 2 weeks.

### 2. Manufacturing of hybrid composites by Ink jet printing

**Production of ink precursors (steps a - c):** Initially gold plates of 30 ml, prepared using beta lactoglobulin fibrils, are concentrated by centrifugation at the rotating speed of 3000 rpm for 15 mins. The gold platelets get sedimented at the bottom of the centrifuge tube and the remaining upper portion of water solution is discarded. Sedimented gold platelets are re-dispersed in the final volume of 3 ml of water solution. The ink is prepared by mixing 2.4 ml of above concentrated gold platelet solution with 0.6 ml of polyethelene glycol (PEG, 5M Da, 2 wt.%). The PEG solution is to adjust the viscosity of the solution and act as humectant, which allows gold platelets to be patterned in air without clogging.

This ink is housed in a syringe attached by luer-lok to a borosilicate 150 µm nozzle. The syringe is pressurized by the air causing the gold platelets to flow down through nozzle.

**Printing** (**step e,** for illustration only): Printing is carried out using a 3- axis micro positioning stage (regen HU, 3D Bio- Printer, Villaz-St-Pierre, Switzerland). The motion is controlled by CAD software. The printing is performed under ambient conditions at 23° C. The printing of "ETH Zurich" logo is done on both glass substrate and white paper. The ink printed on glass substrate is dried using heat gun; the ink printed on paper is dried at ambient conditions.

**Analysis:** The printed letters retain the typical golden appearance of the gold platlets initially used, see fig.6. Accordingly, this ink printing may be used to replace leaf gilding.

Further, the printed gold platelets retain the conductivity behavior. Accordingly, this printed ink can be used as electrodes as well as circuits in the electronics. Conductivity of the printed ink is measured after annealing at 250° C for 2 hours. The annealing procedure removes the organic matrix (i.e. Poly ethylene glycol) in the printed ink. The IV curve after annealing is shown in figure 7, where voltage (V) is given on the X-axis and current (A) is given on the Y-axis. The IV curves shows the ohmic behavior of the printed ink; the material having the conductivity value of 0.056 S.

### 3. Characterization methods for composite materials

3.1: Structural characterization methods for the suspension of single crystal gold platelets and amyloid fibrils of step c:
   ▪ Drop the suspension on glass slips and analyze the size of gold platelets with optical microscopy. The results are shown in Figure 1A
   ▪ Cast the suspension onto a carbon support film on a copper grid, and remove the excess solution after 30 s using a filter paper. The morphologies of amyloid fibrils and gold platelets are analyzed with transmission electron microscopy. The results are shown in Figure 1B and 1C.
3.2 Physical characterization methods for the hybrid composites of step d:
   ▪ The surface is evaluated by visual observation. The results are shown in Fig. 2A.
   ▪ The gold content is analyzed through thermogravimetric analysis at a constant heating rate of 20 °C min⁻¹ from 50 °C to 900 °C under nitrogen protection.
   ▪ Conductivity was determined by using HMS-3000 Hall Measurement System with four-point probes. Current-voltage curves were obtained on 10×10 mm² films to extract the conductivity; results are shown in Fig. 2B.
   ▪ The surfaces of the hybrid composites are evaluated by scanning electron microscopy (shown in Fig. 3A, 3C and 3E) and atomic force microscopy (shown in Fig.3F).
   ▪ The fracture sections of the hybrid composites are observed by scanning electron microscopy. The results are shown in Fig. 3B and 3D.
   ▪ Portions of the films were transferred on glass slides and incubated in sealed glassware at various water activities at 25 °C using saturated salt solutions with known relative humidity: K2SO4 (98.2%), KCl (86.7%) and NaCl (75.7%). The humidity-dependence of the resistance were evaluated and shown in Fig. 4A.
   ▪ Portions of the films were transferred on glass slides and cut into a micro-scratch. The resistance was measured after incubation in 100% humidity and air. The results were shown in Fig. 4B.
   ▪ The films were exposed to water with different pHs. The plasmonic spectra of were acquired in a scattering mode by using a spectrophotometer equipped with a microscope. The results were shown in Fig. 4C.
   ▪ The fluorescence image was obtained with confocal laser-scanning microscopy (CLSM Zeiss 510 Meta). The excitation wavelength was 488 nm and the emission was collected within 518-561 nm. The results are shown in Fig. 4D.

## Claims

1. A composite material consisting of amyloid fibrils and elemental gold, whereby
• said amyloid fibrils are present in the form of 1D nanoscale building blocks and
• said elemental gold is present in the form of 2D nanoscale building blocks and
• said composite material shows a golden shining.

2. The composite material according to claim 1, wherein said amyloid fibrils
(a) have high aspect ratio, preferably with ≤ 10 nm in diameter and ≥ 1µm in length, and / or
(b) have highly charged surfaces, preferably electrophoretic mobilities of the order of 2 µm·cm/V·s at pH 4.

3. The composite material according to any of the preceding claims, wherein said gold
(a) is present as single crystal gold platelets, with the size up to 20 µm and the thickness only of 100 nm or less; and/or
(b) amounts to 10-99 weight-% of the total weight of the composite material.

4. The composite material according to any of the preceding claims, wherein
(a) said elemental gold is present in single crystal platelets and
(b) gold platelets and amyloid fibrils are well-organized into layered structures.

5. The composite material according to claim 1, wherein said gold amounts to 30 - 99% of the total weight of the composite material.

6. A method for manufacturing a composite material according to any of claims 1-5, said method comprising the steps of
(a) growing protein amyloid fibrils by incubating *β-*lactoglobulin solution for 6 h under pH ∼ 2, low ionic strength (I ≤ 20 mM), high temperature (T ∼ 90 °C) and the protein concentration (2 wt.%); and
(b) growing single crystal platelets by mixing 0.67 wt. % amyloid fibrils and 0.066 wt.% chloroauric acid, and then incubating at pH 2 and temperature 60 °C for 12 h; and
(c) optionally concentrating single crystal gold platelets by centrifugation and removing part of upper supernatant amyloid fibrils solution; and then
(f) casting the gold platelets-protein fibrils suspension.

7. A composite material obtainable by, or obtained according to the method of claim 6.

8. The composite material according to claim 7, wherein
(a) said amyloid fibrils were prepared with food-grade proteins; preferably selected from the group consisting of *β*-lactoglobulin, lysozyme, ovalbumin, and serum albumines; and / or
(b) said single crystal gold platelets are prepared by reducing an aqueous solution of gold salts which is stabilized with amyloid fibrils in colloidal state, preferably beta-lactoglobulin in colloidal state.

9. An article comprising a composite material as defined in any of claims 1-5 or claim 7-8.

10. The article of claim 9, selected from the group consisting of
(a) decorative articles, which are partly or fully coated with the composite material;
(b) electrical devices, preferably wires, microdevices or electrical conductors, comprising the composite material; and
(c) sensors comprising the composite material as functional layer or functional element, particularly pH sensors or humidity sensors.

11. Use of the composite material according to any of claims 1-5 or 7-8:
(a) as a coating;
(b) to provide a homogenous metallic gold color, with smooth and shiny surface;
(c) to provide either insulating or metallic; conductivities;
(d) to construct sensing devices to probe the humidity;
(e) to provide a pH sensing property;
(f) to provide fluorescence.

## Patentansprüche

1. Ein Verbundwerkstoff, bestehend aus Amyloidfibrillen und elementarem Gold, wobei
- die Amyloidfibrillen in Form von 1D nanoskaligen Bausteinen vorliegen und
- das elementare Gold in Form von 2D nanoskaligen Bausteinen vorliegt und
- das Verbundmaterial einen goldenen Glanz aufweist.

2. Das Verbundmaterial nach Anspruch 1, wobei die Amyloidfibrillen
(a) ein hohes Aspektverhältnis aufweisen, vorzugsweise mit ≤ 10 nm im Durchmesser und ≥ 1µm in der Länge, und/oder
b) hochgeladene Oberflächen, vorzugsweise elektrophoretische Mobilitäten in der Größenordnung von 2 µm·cm/V·s bei pH 4 aufweisen.

3. Das Verbundmaterial nach einem der vorangehenden Ansprüche, wobei das Gold
(a) als einkristalline Goldplättchen mit einer Größe von bis zu 20 µm und einer Dicke von nur 100 nm oder weniger vorliegt; und/oder
(b) 10-99 Gew.-% des Gesamtgewichts des Verbundwerkstoffs ausmacht.

4. Das Verbundmaterial nach einem der vorangehenden Ansprüche, wobei
(a) das elementare Gold in Einkristallplättchen vorhanden ist und
b) Goldplättchen und Amyloidfibrillen gut in geschichteten Strukturen organisiert sind.

5. Das Verbundmaterial nach Anspruch 1, wobei das Gold 30 - 99% des Gesamtgewichts des Verbundmaterials ausmacht.

6. Ein Verfahren zur Herstellung eines Verbundmaterials nach einem der Ansprüche 1-5, wobei das Verfahren die folgenden Schritte umfasst
(a) Züchten von Proteinamyloidfibrillen durch Inkubieren von β-Lactoglobulinlösung für 6 h bei pH ∼ 2, niedriger Ionenstärke (I ≤ 20 mM), hoher Temperatur (T ∼ 90°C) und der Proteinkonzentration (2 Gew.-%); und
(b) Züchten von Einkristallplättchen durch Mischen von 0,67 Gew.-% Amyloidfibrillen und 0,066 Gew.-% Chloroaursäure und dann Inkubieren bei pH 2 und Temperatur 60 °C für 12 h; und
(c) gegebenenfalls Konzentrieren von einkristallinen Goldplättchen durch Zentrifugieren und Entfernen eines Teils der Lösung der oberen überstehenden Amyloidfibrillen; und dann
(f) Gießen der Goldplättchen-Protein-Fibrillen-Suspension.

7. Ein Verbundwerkstoff, erhältlich durch oder erhalten nach dem Verfahren nach Anspruch 6.

8. Das Verbundmaterial nach Anspruch 7, wobei
(a) die Amyloidfibrillen mit Proteinen von Lebensmittelqualität hergestellt wurden; vorzugsweise ausgewählt aus der Gruppe bestehend aus β-Lactoglobulin, Lysozym, Ovalbumin und Serumalbumin; und / oder
(b) die einkristallinen Goldplättchen durch Reduzieren einer wässrigen Lösung von Goldsalzen hergestellt werden, die mit Amyloidfibrillen in kolloidalem Zustand stabilisiert ist, vorzugsweise Beta-Lactoglobulin in kolloidalem Zustand.

9. Ein Gegenstand, umfassend ein Verbundmaterial nach einem der Ansprüche 1-5 oder 7-8.

10. Der Gegenstand nach Anspruch 9, ausgewählt aus der Gruppe bestehend aus
a) Dekorationsartikel, die teilweise oder vollständig mit dem Verbundwerkstoff beschichtet sind;
(b) elektrische Vorrichtungen, vorzugsweise Drähte, Mikrovorrichtungen oder elektrische Leiter, die das Verbundmaterial umfassen; und
(c) Sensoren, die das Verbundmaterial als Funktionsschicht oder Funktionselement umfassen, insbesondere pH-Sensoren oder Feuchtigkeitssensoren.

11. Verwendung des Verbundwerkstoffs nach einem der Ansprüche 1-5 oder 7-8:
(a) als Beschichtung;
b) um eine homogene metallische Goldfarbe mit glatter und glänzender Oberfläche zu erhalten;
c) um entweder isolierende oder metallische Leitfähigkeiten bereitzustellen;
(d) zum Konstruieren von Sensorvorrichtungen zum Abtasten der Feuchtigkeit;
(e) um eine pH-Messeigenschaft bereitzustellen;
(f) um Fluoreszenz bereitzustellen.

## Revendications

1. Un matériau composite constitué de fibrilles amyloïdes et d'or élémentaire, dans lequel
- lesdites fibrilles amyloïdes sont présentes sous la forme de blocs de construction à l'échelle nanométrique 1D et
- ledit or élémentaire est présent sous la forme de blocs de construction à l'échelle nanométrique 2D et
- ledit matériau composite présente une brillance dorée.

2. Matériau composite selon la revendication 1, dans lequel lesdites fibrilles amyloïdes
(a) ont un rapport d'aspect élevé, de préférence avec ≤ 10 nm de diamètre et ≥ µm de longueur, et / ou
b) présentent des surfaces très chargées, de préférence des mobilités électrophorétiques de l'ordre de 2 µm·cm/V·s à pH 4.

3. Le matériau composite selon l'une quelconque des revendications précédentes, dans lequel ledit or
a) est présent sous forme de plaquettes d'or monocristallin, d'une taille allant jusqu'à 20 µm et d'une épaisseur égale ou inférieure à 100 nm; et/ou
b) représente 10 à 99 % en poids du poids total du matériau composite.

4. Le matériau composite selon l'une quelconque des revendications précédentes, dans lequel
(a) ledit or élémentaire est présent dans les plaquettes monocristallines et
b) les plaquettes d'or et les fibrilles amyloïdes sont bien organisées en couches.

5. Le matériau composite selon la revendication 1, dans lequel ledit or représente 30 à 99 % du poids total du matériau composite.

6. Un procédé de fabrication d'un matériau composite selon l'une quelconque des revendications 1 à 5, ledit procédé comprenant les étapes consistant à
(a) cultiver de fibrilles amyloïdes protéiques par incubation de la solution de β-lactoglobuline pendant 6 h sous pH ∼ 2, faible force ionique (I ≤ 20 mM), haute température (T ∼ 90 °C) et concentration en protéines (2 % en poids) ; et
(b) cultiver de plaquettes monocristallines en mélangeant 0,67 % en poids de fibrilles amyloïdes et 0,066 % en poids d'acide chloroaurique, puis en incubant à pH 2 et à 60 °C pendant 12 h ; et
c) concentrer optionnellement de plaquettes d'or monocristallin par centrifugation et élimination d'une partie de la solution de fibrilles amyloïdes surnageantes supérieures; et ensuite
f) couler la suspension de plaquettes d'or et de fibrilles protéiques.

7. Un matériau composite pouvant être obtenu par, ou obtenu selon le procédé de la revendication 6.

8. Le matériau composite selon la revendication 7, dans lequel
(a) lesdites fibrilles amyloïdes ont été préparées avec des protéines de qualité alimentaire; de préférence choisies dans le groupe constitué de β-lactoglobuline, lysozyme, ovalbumine et sérum albumine; et / ou
(b) lesdites plaquettes d'or monocristallin sont préparées en réduisant une solution aqueuse de sels d'or qui est stabilisée avec des fibrilles amyloïdes à l'état colloïdal, de préférence une bêta-lactoglobuline à l'état colloïdal.

9. Un article comprenant un matériau composite tel que défini dans l'une quelconque des revendications 1-5 ou 7-8.

10. L'article selon la revendication 9, choisi dans le groupe constitué par
a) des articles de décoration qui sont partiellement ou totalement recouverts du matériau composite;
b) des dispositifs électriques, de préférence des fils, des microdispositifs ou des conducteurs électriques, comprenant le matériau composite; et
c) des capteurs comprenant le matériau composite comme couche fonctionnelle ou élément fonctionnel, en particulier capteurs de pH ou capteurs d'humidité.

11. Utilisation du matériau composite selon l'une quelconque des revendications 1-5 ou 7-8:
a) comme revêtement;
b) pour obtenir une couleur dorée métallique homogène, avec une surface lisse et brillante;
c) pour fournir des conductivités soit isolantes, soit métalliques;
d) pour construire des dispositifs de détection pour sonder l'humidité;
e) pour fournir une propriété de détection du pH;
f) pour fournir une fluorescence.
